# EUROPEAN PATENT SPECIFICATION

(11) **EP 3 686 897 B1**
(45) Date of publication and mention of the grant of the patent: **22.05.2024**
(21) Application number: 19153273.8
(22) Date of filing: 23.01.2019
(51) Int. Cl.: G16H 50/30

(54) **METHOD AND DATA PROCESSING UNIT FOR SELECTING A RISK ASSESSMENT COMPUTER PROGRAM**
VERFAHREN UND DATENVERARBEITUNGSEINHEIT ZUR AUSWAHL EINES RISIKOBEWERTUNGSCOMPUTERPROGRAMMS
PROCÉDÉ ET UNITÉ DE TRAITEMENT DE DONNÉES DE SÉLECTION D'UN PROGRAMME INFORMATIQUE D'ÉVALUATION DU RISQUE

(43) Date of publication of application: 29.07.2020
(73) Proprietor: Siemens Healthineers AG, 91301 Forchheim (DE)
(72) Inventor: Costa, Maria Jimena, 90482 Nuernberg (DE); Heissner, Klaus, 91054 Erlangen (DE); Jattke, Kirstin, 90482 Nürnberg (DE); Kamen, Ali, Skillman, 08558 (US)
(74) Representative: Siemens Healthineers Patent Attorneys

(56) References cited:
- US-A1- 2016 357 934
- C. LEE VENTOLA: "Mobile Devices and Apps for Health Care Professionals: Uses and Benefits", P & T : A PEER-REVIEWED JOURNAL FOR FORMULARY MANAGEMENT, vol. 39, no. 5, 31 May 2014 (2014-05-31), pages 356-364, XP055599459,
- Anonymous: "Cloud storage - Wikipedia", , 16 January 2019 (2019-01-16), XP055600575, Retrieved from the Internet: URL:https://en.wikipedia.org/w/index.php?t itle=Cloud_storage&oldid=878764684 [retrieved on 2019-06-28]

## Description

The invention relates in one aspect to a computer-implemented method for displaying a graphical user interface that contains a selection element to select one risk assessment computer program out of a patient-related subset of a plurality of risk assessment computer programs of a patient on a display unit for an user. In another aspect, the invention relates to a data processing unit, a computer program product and a computer-readable medium. The scope of the invention is defined by the appended claims 1-9.

In the decision-making process within a hospital environment typically risk assessment computer programs are used by a physician to decide on the management of a patient. Such a risk assessment computer program can be an essential tool for the physician because it usually provides an accurate estimate of risk. Risk assessment computer programs can be used for designing a clinical trial, e.g., to ensure that homogeneous and/or high-risk patient groups are included. One advantage of said risk assessment computer programs can be the increased performance in predicting probabilities of clinical outcome of the managed patient compared to clinical judgment of the physician. The risk assessment computer programs are typically used at different points in the patient's evolution and/or clinical pathway, for instance, early to predict cancer on initial biopsy and/or on repeat biopsy, or at a later timepoint to predict biochemical recurrence after specific treatment of the cancer.

Usually over 100 different risk assessment computer programs are available to the physician and/or provided within a computer network, e.g. the Internet. However, the proper selection of the risk assessment computer program suitable at that specific point in the patient's evolution and/or clinical pathway can be very challenging for the physician.

US 2016 / 0 357 934 A1 discloses a system and method to facilitate predicting the onset of diabetes in a non-diabetic population or symptom progression in those patients already suffering from the disease. In US 2016 / 0 357 934 A1, different data models used for the prediction are generated based on population data and the best models to be applied to different segments of the population data are identified. US 2016 / 0 357 934 A1 does not concern the features related to calculability of risk assessment computer programs nor the features related to displaying the differentiated patient-related subset of the plurality of risk assessment computer programs according to their calculability on the display unit for the user. C. Lee Ventola describes in "Mobile Devices and Apps for Health Care Professionals: Uses and Benefits", P & T, vol. 39, no. 5, 31 May 2014, pages 356-364, XP055599459, different computer programs, in particular mobile apps, available for health care professionals.

The underlying technical problem of the invention is to provide a computer-implemented method for displaying a graphical user interface that contains a selection element to select one risk assessment computer program out of a patient-related subset of a plurality of risk assessment computer programs of a patient on a display unit for an user, a data processing unit, a computer program product and a computer-readable medium with increased usability for the physician.

This problem is solved by the features of the independent claims. Advantageous embodiments are disclosed within the dependent claims.

The invention relates in one aspect to a computer-implemented method for displaying a graphical user interface that contains a selection element to select one risk assessment computer program out of a patient-related subset of a plurality of risk assessment computer programs on a display unit for an user, the method comprising:
- retrieving a set of disease-related workflows, each disease-related workflow describing a clinical pathway and/or a patient's evolution with regard to a particular disease and comprising at least two disease-related stages and being stored within a computer network, the at least two disease-related stages being part of such clinical pathway and/or patient's evolution with regard to said particular disease,
- retrieving a plurality of risk assessment computer programs, each risk assessment computer program predicting a probability of clinical outcome as the function of values in input parameter categories and being stored within the computer network,

- retrieving a patient-related data record of the patient from the computer network,
- selecting a disease-related dataset from the patient-related data record,
- determining at least one of the disease-related workflow stages from the set of disease-related workflows based on a first disease-related mapping function, the selected disease-related dataset being an input of the first disease-related mapping function,
- providing a set of said input parameter categories,
- selecting a patient-related dataset from the patient-related data record, wherein the patient-related dataset comprises at least one input parameter category,
- comparing the patient-related dataset with the provided set of the input parameter categories of the plurality of risk assessment computer programs, whereby a calculable subset of the plurality of risk assessment computer programs is determined,
- determining a patient-related subset of the plurality of risk assessment computer programs based on a second disease-related mapping function, the determined at least one of the disease-related workflow stage and the calculable subset being an input of the second disease-related mapping function, wherein the patient-related subset of the plurality of risk assessment computer programs is differentiated by the determined calculable subset, and
- displaying the graphical user interface that contains the selection element to select said one risk assessment computer program out of the patient-related subset of the plurality of risk assessment computer programs of the patient on the display unit for the user, wherein the displaying the graphical user interface comprises displaying the differentiated patient-related subset of the plurality of risk assessment computer programs according to their calculability on the display unit for the user.

This aspect of the invention is particularly advantageous since in addition to the at least one of the disease-related workflow stages the calculability of the plurality of risk assessment computer programs is considered. Thereby typically the time and/or effort for the user is further reduced. One further advantage of this aspect of the invention is the visual depiction of the calculability of the respective risk assessment computer program which usually reduces the time and/effort for assessing this information. The patient-related subset can be particularly split into a calculable subset and a non-calculable subset of risk assessment computer programs.

The invention relates in another aspect to a computer-implemented method for displaying a graphical user interface that contains a selection element to select one risk assessment computer program out of a patient-related subset of a plurality of risk assessment computer programs on a display unit for an user, the method comprising:
- retrieving a set of disease-related workflows, each disease-related workflow describing a clinical pathway and/or a patient's evolution with regard to a particular disease and comprising at least two disease-related stages and being stored within a computer network, the at least two disease-related stages being part of such clinical pathway and/or patient's evolution with regard to said particular disease,
- retrieving a plurality of risk assessment computer programs, each risk assessment computer program predicting a probability of clinical outcome as the function of values in input parameter categories and being stored within the computer network,
- retrieving a patient-related data record of the patient from the computer network,
- selecting a disease-related dataset from the patient-related data record,
- determining at least one of the disease-related workflow stages from the set of disease-related workflows based on a first disease-related mapping function, the selected disease-related dataset being an input of the first disease-related mapping function,
- providing a set of said input parameter categories,
- selecting a patient-related dataset from the patient-related data record, wherein the patient-related dataset comprises at least one input parameter category,
- comparing the patient-related dataset with the provided set of the input parameter categories of the plurality of risk assessment computer programs, whereby a calculable subset of the plurality of risk assessment computer programs is determined,
- determining a patient-related subset of the plurality of risk assessment computer programs based on a second disease-related mapping function, the determined at least one of the disease-related workflow stage and the calculable subset being an input of the second disease-related mapping function, wherein the patient-related subset of the plurality of risk assessment computer programs is merged with the determined calculable subset, wherein merging comprises an AND-operation and wherein a calculable patient-related subset of the plurality of risk assessment computer programs is determined, and
- displaying the graphical user interface that contains the selection element to select said one risk assessment computer program out of the calculable patient-related subset of the plurality of risk assessment computer programs of the patient on the display unit for the user.

An advantage of the another aspect of the invention is that typically only such risk assessment computer programs are selected and/or executed that are also calculable.

The aspects of the inventions are in particular advantageous for following reasons:
Displaying the patient-related subset of the plurality of risk assessment computer programs reduce effort and/or time for the user to select one appropriate risk assessment computer program. Thereby typically also costs are reduced. Alternatively, or additionally a management of the patient can be improved because the displaying the graphical user interface is standardized. Another advantage can be an increased repeatability and/or reproducibility at managing the patient. The patient-related subset of the plurality of risk assessment computer programs comprises preferably such risk assessment computer programs which are relevant for the patient and/or for the particular point in the patient's evolution and/or clinical pathway.

One additional advantage of the method can be that the patient can be assigned to specific stages of disease-related workflows, so a decision-making process could be sped up. The method can be beneficial because it provides a possibility to handle a wide range (N > 100) of different risk assessment computer programs easily by the user. The user can be a physician and/or a health professional.

The computer network comprises typically at least one server computer and one client computer. Usually the computer network comprises a memory unit for storing data and/or signals. The server computer can be part of a hospital network that is accessible via the Internet or is disconnected from the Internet. The server computer can be a virtual machine within another host server computer. The server computer can be physically located within a data center wherein the data center is connected to the hospital network and/or to the Internet. The client computer is typically connected to the hospital network and/or to the Internet. Usually, the client computer and the server computer have a network interface for communicating the signals and/or the data. The communicating can comprise sending and/or retrieving said signals and/or data. The retrieving the set of disease-related workflows, the plurality of risk assessment computer programs and/or the patient-related data record occurs typically by using or via the network interface. The signals and/or data are typically sent from the server computer and retrieved by the client computer or vice versa. The retrieving can comprise a downloading and/or an uploading and/or a storing of the signals and/or the data. The data can comprise said risk assessment computer programs.

Basically, the client computer and the server computer can be physically installed within a single host server computer. In that case, the client computer and the server computer are part of the single host server computer. The client computer, the server computer and/or the host server computer comprises typically a RAM unit, a memory unit, a data processing unit and/or the network interface. The client computer can comprise said display unit. The client computer can be a stationary computer and/or a portable computer such as a smartphone and/or tablet computer. The network interface comprises usually an encryption module for encrypting the signals and/or data.

The predicted probability of clinical outcome of each risk assessment computer program correlates typically to a risk for the patient. The risk for the patient can comprise a risk for initial occurrence, a risk of recurrence and/or a risk of death. Basically, a lots of different risk types can be assessed for different types of diseases. For example, Shariat et al. describe already a set of risk assessment computer programs in the specific field of prostate cancer ("An Updated Catalog of Prostate Cancer Predictive Tools", DOI 10.1002/cncr.23908). The predicted probability of clinical outcome can be used to establish a diagnosis and/or prognosis for the patient. The plurality of risk assessment computer programs may comprise less than 100 computer programs, about 100 computer programs and/or more than 100 computer programs.

The risk assessment computer programs are in general an array of risk assessment formulas for various stages of treatment and/or diagnosis and/or various clinical questions. Each risk assessment computer program can be typically assigned to at least one of the following types:
- nomogram,
- risk grouping,
- probability table,
- classification tree,
- regression tree,
- artificial network.

The set of disease-related workflows comprises typically more than one disease-related workflow. Each disease-related workflow describes typically at least one clinical pathway of the following:
- cancer,
- cardiac disease,
- lung disease,
- anatomical disease,
- blood disease.

The disease-related workflows are typically established based on technical literature and/or clinical experience by the user. Every disease-related workflow describes a clinical pathway and/or a patient's evolution with regard to a particular disease. The patient suffers usually from that particular disease. The at least two disease-related stages are part of such clinical pathway and/or patient's evolution with regard to said particular disease. The at least two disease-related stages are usually discrete stages. The at least two disease-related stages can be typically uniquely assigned to the disease-related workflows. The clinical pathway can comprise different temporal and/or local, in particular subsequent, steps of treating and/or diagnosing the patient. The patient's evolution usually comprises different phases of a progression of the disease from the patient's perspective. Such phases can be definable from low risk early stage to advance post treatment recurrence. The phases are typically discrete. The disease-related workflows are usually part of disease-related workflow computer programs and/or part of disease-related workflow rule sets that can in particular be stored within and/or retrieved from the computer network.

The patient-related data record of the patient can be stored within a picture archiving and communication system and/or a radiology information system and/or a patient information system, said systems typically being part of and/or connected to the computer network. The patient-related data record comprises usually the clinical and/or medical history and/or protected health information of the patient. The protected health information can comprise social security number, first name, last name and/or birth date of the patient.

Usually the patient-related data record can be classified into a disease-related dataset and into other data. The other data can comprise the protected health information. The disease-related dataset comprises the clinical and/or medical history, optionally in addition the age of the patient. The clinical and/or medical history comprises at least one of the following:
- diagnostic findings,
- diagnostic images,
- therapeutic results.

Optionally, additionally at least one of the following:
- genomics,
- comorbidities,
- demographic data.

The selecting the disease-related dataset may comprise said classifying. The selecting is usually conducted automatically based on a predefined rule set. The predefined rule set can be defined by the user and/or the technical literature. Basically, the selecting can be conducted by the user, for instance using the display unit. Typically, only the disease-related dataset is selected from the patient-related data record.

The determining by a disease-related mapping function typically has an effect similar to filtering, ranking and/or classifying.

The determining the at least one of the disease-related workflow stages occurs typically automatically after selecting the disease-related dataset. The at least one of the disease-related workflow stages from the set of disease-related workflows is typically the output having applied the first disease-related mapping function on the selected disease-related dataset. By determining the at least one of the disease-related workflow stages typically the disease-related dataset is linked to the set of disease-related workflows, in particular to the at least two disease-related stages. The linking can comprise a linking probability representing usually how likely the patient is at said at least one of the disease-related workflow stages. Usually only one disease-related workflow stage is determined. Alternatively, or additionally a group of disease-related workflow stages can be determined by the first disease-related mapping function wherein said group typically comprises similar and/or adjacent disease-related workflow stages.

The determining the patient-related subset of the plurality of risk assessment computer programs is conceptually similar to the preceding act of determining the at least one of the disease-related workflow stages, basically differentiating in the input and output. Usually the patient-related subset comprises more than one of the plurality of risk assessment computer programs.

The graphical user interface is displayed on the display unit for the user. The display unit can comprise a screen, a monitor and/or an input device such as keyboard, mouse, and/or voice control. By displaying the graphical user interface containing the selection element to select one risk assessment computer program out of a patient-related subset of the plurality of risk assessment computer programs of the patient, the selection element is typically provided for the user to select said risk assessment computer program, especially by looking at the display unit and/or using the input device.

The selection element can comprise a selection list that contains the patient-related subset of the plurality of risk assessment computer programs. The selection list is preferably sortable by the user according to relevance with respect to urgency and/or efficacy and/or precision of the patient-related subset of the plurality of risk assessment computer programs. The selection element can comprise a checkbox next to and/or a toggle button of the item being part of the patient-related subset of the plurality of risk assessment computer programs. The display unit preferably comprises a speech recognition system configured to select said item by voice control if the user wants to select said one risk assessment computer program out of the patient-related subset of the plurality of risk assessment computer programs of the patient on the display unit for the user. Alternatively, and or additionally the displaying the graphical user interface comprises displaying the non-patient-related subset of the plurality of risk assessment computer programs wherein the non-patient-related subset is highlighted differently compared to the patient-related subset on the display unit.

Said risk assessment computer programs are usually provided as packed or compiled computer programs, e.g. using 7z-, zip- , exe- and/or msi-packaging. The risk assessment computer programs are typically executable on the server computer and/or the client computer. If executed on the server computer, for instance in the virtual machine, the network interface is usually configured to stream the execution of said executed risk assessment computer program to the client computer. Alternatively, or additionally the risk assessment computer programs can be executed directly on the client computer. The client computer, the host server computer and/or the server computer typically comprise at least one of the following operating systems: Windows, Linux, Unix, Android, iOS, macOS, etc. Typically, the risk assessment computer programs are adapted to the appropriate operating system.

The selected one risk assessment computer program out of the patient-related subset of the plurality of risk assessment computer programs of the patient can be executed before, during and/or after displaying the graphical user interface that contains the selection element to select said one risk assessment computer program out of the patient-related subset of the plurality of risk assessment computer programs of the patient on the display unit for the user. Advantageously can be the execution before and/or during the displaying since the user can access or apply the selected risk assessment computer program typically quicker compared to delayed execution. In other words, the user typically uses or applies the selected risk assessment computer program after said displaying of the graphical user interface. Having selected said one risk assessment computer program the user and/or another user can use or apply it for managing the patient, in particular for assessing the predicted probability of clinical outcome.

The input parameter categories are at least one type of the following:
- blood test,
- initial biopsy,
- molecular test,
- age.

The set of input parameter categories can be provided by retrieving the plurality of risk assessment computer programs. The set of input parameter categories are typically defined by identifying input values of the plurality of risk assessment computer programs. In other words, the set of input parameter categories can be a set of program variables. Typically, every risk assessment computer program depends at least on one input parameter category or program variable. Usually, the patient-related data record comprises at least one input parameter categories of the set of input parameter categories and/or its corresponding result or value. Typically, the patient-related data record can be clustered or classified according to the set of input parameter categories. Usually, the clinical and/or medical history, in particular the selected disease-related dataset, comprises at least one input parameter category, especially a result or a value of such at least one input parameter category. Typically, another patient-related dataset without any input parameter category cannot be selected.

The comparing the patient-related dataset with the provided set of input parameter categories comprises identifying such risk assessment computer programs that are calculable using the selected disease-related dataset or using the patient-related data record. The input based on the patient-related dataset for the determining the at least one of the disease-related workflow stages can differ from the input based on the patient-related dataset for comparing the patient-related dataset with the provided set of input parameter categories. The comparing comprises basically matching the input parameter categories of the patient-related data record, in particular the selected disease-related dataset, with the input parameter categories of the plurality of risk assessment computer programs and assigning such risk assessment computer programs to the calculable subset that can be calculated using the input parameter categories of the patient-related data record, in particular the selected disease-related dataset. In other words, the calculable subset comprises those risk assessment computer programs that can be calculated without additional tests on or of the patient. A risk assessment computer program is calculable, if an output on basis of the required input parameter categories can be generated upon execution.

One embodiment of the invention relates in one aspect to a method, wherein the displaying the graphical user interface comprises identifying such input parameter categories of the patient-related subset of the plurality of risk assessment computer programs, missing for the calculability, and displaying the identified missing input parameter categories on the display unit for the user. This embodiment is particularly beneficial for the user since by considering the missing input parameter categories the patient can be correspondingly examined. Typically, at such examination the result and/or value of such missing input parameter category can be determined and added to the patient-related data record. In that case, usually the formerly non-calculable risk assessment computer program can be assigned to the calculable subset if the input parameter categories match primarily.

One embodiment of the invention relates in one aspect to a method, wherein the first disease-related mapping function is determined by training a machine learning system based on a first set of training data and/or wherein the second disease-related mapping function is determined by training the machine learning system based on a second set of training data. The training of the first disease-related mapping function and/or the second disease-related mapping function using the machine learning system is beneficial because such machine learning system can be specifically tailored to patients.

One embodiment of the invention relates in one aspect to a method, wherein the machine learning system and/or the first disease-related mapping function and/or the second disease-related mapping function is based on an artificial neural network. The artificial neural network can be advantageously executed within the computer network and/or the client computer and/or the host server computer and/or the server computer.

One embodiment of the invention relates in one aspect to a method, wherein the artificial neural network comprises a convolutional neural network. The convolutional neural network is typically very adaptable and/or efficient. The convolution neural network comprises in particular an input layer, an output layer, and an intermediate layer, said layers connected via weighted links. The training of such convolutional neural network typically comprises determining the weights of the links.

The data processing unit can be realized as a data processing system or as a part of a data processing system. The data processing system can, for example, comprise cloud-computing system, a computer network, a computer, a tablet computer, a smartphone or the like. The cloud-computing system is typically connected via the Internet. The data processing system can comprise hardware and/or software. The hardware can be, for example, a processor system, a memory system and combinations thereof. The hardware can be configurable by the software and/or be operable by the software.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

Reference is made to the fact that the described methods and the described data processing unit as well as the described imaging device are merely preferred example embodiments of the invention and that the invention can be varied by a person skilled in the art, provided it is as specified by the claims. Said features, advantages or alternative embodiments of the apparatus apply also to the method and vice-versa, as long as they are as specified by the claims.

The invention will be illustrated below with reference to the accompanying figures using example embodiments. The illustration in the figures is schematic and highly simplified and not necessarily to scale.
Fig. 1 shows a diagram illustrating a method according to one aspect of the invention.
Fig. 2 shows a diagram illustrating a method according to another aspect of the invention.
Fig. 3 shows a diagram illustrating a method according to a further aspect of the invention.

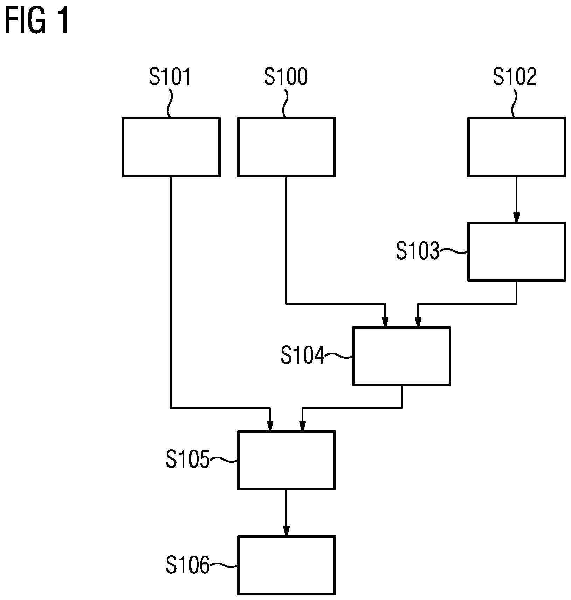

**Fig. 1** shows a diagram illustrating a computer-implemented method for displaying a graphical user interface that contains a selection element to select one risk assessment computer program out of a patient-related subset of a plurality of risk assessment computer programs of a patient on a display unit for an user.

Method step S100 includes retrieving a set of disease-related workflows, each disease-related workflow comprising at least two disease-related stages and being stored within a computer network.

Method step S101 includes retrieving a plurality of risk assessment computer programs, each risk assessment computer program predicting a probability of clinical outcome and being stored within the computer network.

Method step S102 includes retrieving a patient-related data record of the patient from the computer network.

Method step S103 includes selecting a disease-related dataset from the patient-related data record.

Method step S104 includes determining at least one of the disease-related workflow stages from the set of disease-related workflows based on a first disease-related mapping function, the selected disease-related dataset being an input of the first disease-related mapping function.

Method step S105 includes determining a patient-related subset of the plurality of risk assessment computer programs based on a second disease-related mapping function, the determined at least one of the disease-related workflow stage being an input of the second disease-related mapping function.

Method step S106 includes displaying the graphical user interface that contains the selection element to select said one risk assessment computer program out of the patient-related subset of the plurality of risk assessment computer programs of the patient on the display unit for the user.

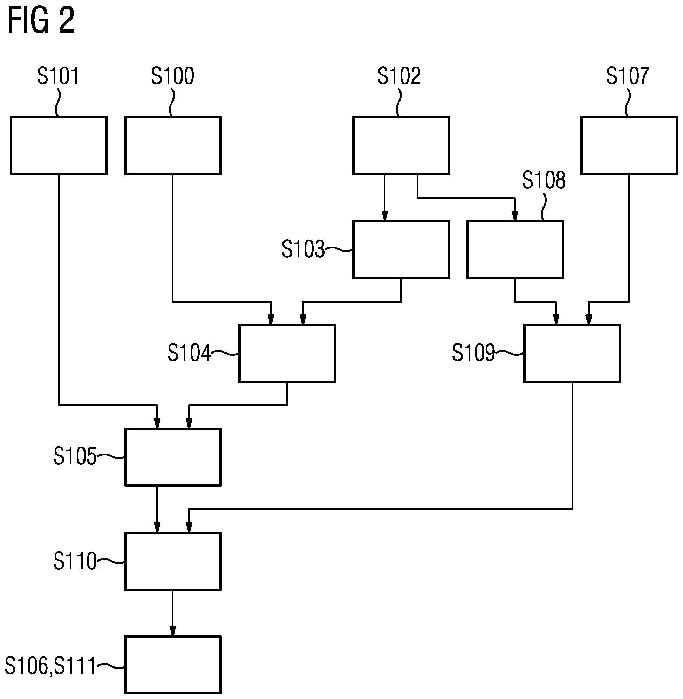

**Fig. 2** shows a diagram illustrating a method according to another aspect of the invention.

This aspect comprises method steps S100 to S106 as shown in Fig. 1. Additionally, a calculable subset of the plurality of risk assessment computer programs is determined, wherein the calculable subset is used as input for determining the patient-related subset and wherein the determining the calculable subset comprises following steps:
Method step S107 includes providing a set of input parameter categories, wherein the plurality of risk assessment computer programs depends on the set of input parameter categories, each risk assessment computer program predicting the probability of clinical outcome as the function of values in said input parameter categories.
Method step S108 includes selecting a patient-related dataset from the patient-related data record, wherein the patient-related dataset comprises at least one input parameter category.
Method step S109 includes comparing the patient-related dataset with the provided set of input parameter categories of the plurality of risk assessment computer programs, whereby the calculable subset of the plurality of risk assessment computer programs is determined.
Method step S110 includes that the patient-related subset of the plurality of risk assessment computer programs is differentiated by the determined calculable subset and wherein the displaying the graphical user interface comprises displaying the differentiated patient-related subset of the plurality of risk assessment computer programs according to their calculability on the display unit for the user.
Method step Sill includes that the displaying the graphical user interface comprises identifying such input parameter categories of the patient-related subset of the plurality of risk assessment computer programs, missing for the calculability, and displaying the identified missing input parameter categories on the display unit for the user.

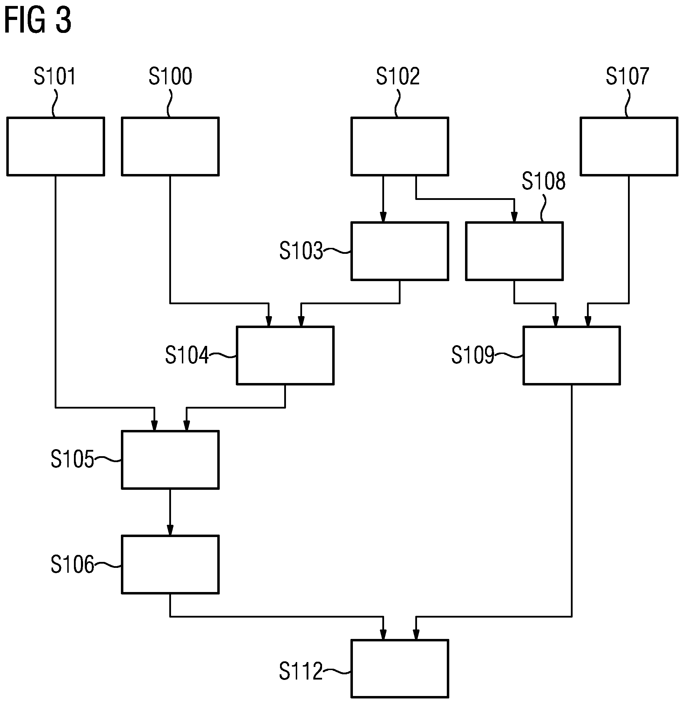

**Fig. 3** shows a diagram illustrating a method according to another aspect of the invention.

This aspect comprises method steps S100 to S109 as shown in Fig. 1 and Fig. 2.

The method step S112 includes that the patient-related subset of the plurality of risk assessment computer programs is merged with the determined calculable subset, wherein a calculable patient-related subset of the plurality of risk assessment computer programs is determined, and wherein the graphical user interface is configured such that it contains the selection element to select said one risk assessment computer program out of the calculable patient-related subset of the plurality of risk assessment computer programs of the patient on the display unit for the user.

## Claims

1. A computer-implemented method for displaying a graphical user interface that contains a selection element to select one risk assessment computer program out of a patient-related subset of a plurality of risk assessment computer programs on a display unit for an user, the method comprising:
- retrieving (S100) a set of disease-related workflows, each disease-related workflow describing a clinical pathway and/or a patient's evolution with regard to a particular disease and comprising at least two disease-related stages and being stored within a computer network, the at least two disease-related stages being part of such clinical pathway and/or patient's evolution with regard to said particular disease,
- retrieving (S101) a plurality of risk assessment computer programs, each risk assessment computer program predicting a probability of clinical outcome as the function of values in input parameter categories and being stored within the computer network,
- retrieving (S102) a patient-related data record of the patient from the computer network,
- selecting (S103) a disease-related dataset from the patient-related data record,
- determining (S104) at least one of the disease-related workflow stages from the set of disease-related workflows based on a first disease-related mapping function, the selected disease-related dataset being an input of the first disease-related mapping function,
- providing (S107) a set of said input parameter categories,
- selecting (S108) a patient-related dataset from the patient-related data record, wherein the patient-related dataset comprises at least one input parameter category,
- comparing (S109) the patient-related dataset with the provided set of the input parameter categories of the plurality of risk assessment computer programs, whereby a calculable subset of the plurality of risk assessment computer programs is determined,
- determining (S105) a patient-related subset of the plurality of risk assessment computer programs based on a second disease-related mapping function, the determined at least one of the disease-related workflow stage and the calculable subset being an input of the second disease-related mapping function, wherein (S110) the patient-related subset of the plurality of risk assessment computer programs is differentiated by the determined calculable subset, and
- displaying (S106) the graphical user interface that contains the selection element to select said one risk assessment computer program out of the patient-related subset of the plurality of risk assessment computer programs of the patient on the display unit for the user, wherein the displaying the graphical user interface comprises displaying the differentiated patient-related subset of the plurality of risk assessment computer programs according to their calculability on the display unit for the user.

2. The method of claim 1, wherein the displaying the graphical user interface comprises identifying (Sill) such input parameter categories of the patient-related subset of the plurality of risk assessment computer programs, missing for the calculability, and displaying the identified missing input parameter categories on the display unit for the user.

3. A computer-implemented method for displaying a graphical user interface that contains a selection element to select one risk assessment computer program out of a patient-related subset of a plurality of risk assessment computer programs on a display unit for an user, the method comprising:
- retrieving (S100) a set of disease-related workflows, each disease-related workflow describing a clinical pathway and/or a patient's evolution with regard to a particular disease and comprising at least two disease-related stages and being stored within a computer network, the at least two disease-related stages being part of such clinical pathway and/or patient's evolution with regard to said particular disease,
- retrieving (S101) a plurality of risk assessment computer programs, each risk assessment computer program predicting a probability of clinical outcome as the function of values in input parameter categories and being stored within the computer network,
- retrieving (S102) a patient-related data record of the patient from the computer network,
- selecting (S103) a disease-related dataset from the patient-related data record,
- determining (S104) at least one of the disease-related workflow stages from the set of disease-related workflows based on a first disease-related mapping function, the selected disease-related dataset being an input of the first disease-related mapping function,
- providing (S107) a set of said input parameter categories,
- selecting (S108) a patient-related dataset from the patient-related data record, wherein the patient-related dataset comprises at least one input parameter category,
- comparing (S109) the patient-related dataset with the provided set of the input parameter categories of the plurality of risk assessment computer programs, whereby a calculable subset of the plurality of risk assessment computer programs is determined,
- determining (S105) a patient-related subset of the plurality of risk assessment computer programs based on a second disease-related mapping function, the determined at least one of the disease-related workflow stage and the calculable subset being an input of the second disease-related mapping function, wherein (S112) the patient-related subset of the plurality of risk assessment computer programs is merged with the determined calculable subset, wherein merging comprises an AND-operation and wherein a calculable patient-related subset of the plurality of risk assessment computer programs is determined, and
- displaying (S106) the graphical user interface that contains the selection element to select said one risk assessment computer program out of the calculable patient-related subset of the plurality of risk assessment computer programs of the patient on the display unit for the user.

4. The method of any one of the preceding claims,
- wherein the first disease-related mapping function is determined by training a machine learning system based on a first set of training data and/or
- wherein the second disease-related mapping function is determined by training the machine learning system based on a second set of training data.

5. The method of claim 4, wherein the machine learning system and/or the first disease-related mapping function and/or the second disease-related mapping function is based on an artificial neural network.

6. The method of claim 5, wherein the artificial neural network comprises a convolutional neural network.

7. A data processing unit, comprising:
- a processor,
- a network interface,
- the display unit,
wherein the data processing is configured for performing the method of any one of claims 1 to 6.

8. A computer program product comprising a computer program, the computer program being loadable into a memory unit of a data processing system, including program code sections to make the data processing system execute the method of any one of claims 1 to 6 when the computer program is executed in said data processing system.

9. A computer-readable medium, on which program code sections of a computer program are saved, said program code sections being loadable into and/or executable in a data processing system to make the data processing system execute the method of any one of the claims 1 to 6 when the program code sections are executed in said data processing system.

## Patentansprüche

1. Computerimplementiertes Verfahren zum Anzeigen einer grafischen Benutzeroberfläche, die ein Auswahlelement zum Auswählen eines Computerprogramms zur Risikobewertung aus einer patientenbezogenen Teilmenge einer Mehrzahl von Computerprogrammen zur Risikobewertung enthält, auf einer Anzeigeeinheit für einen Benutzer, wobei das Verfahren umfasst:
- Abrufen (S100) eines Satzes von krankheitsbezogenen Arbeitsflüssen, wobei jeder krankheitsbezogene Arbeitsfluss einen klinischen Verlauf und/oder eine Entwicklung eines Patienten hinsichtlich einer spezifischen Krankheit beschreibt und mindestens zwei krankheitsbezogene Phasen umfasst und innerhalb eines Computernetzwerks gespeichert wird, wobei die mindestens zwei krankheitsbezogenen Phasen Teil solch eines klinischen Verlaufs und/oder solch einer Entwicklung eines Patienten hinsichtlich der spezifischen Krankheit sind,
- Abrufen (S101) einer Mehrzahl von Computerprogrammen zur Risikobewertung, wobei jedes Computerprogramm zur Risikobewertung eine Wahrscheinlichkeit eines klinischen Ergebnisses in Abhängigkeit von Werten in Eingabeparameterkategorien vorhersagt und innerhalb des Computernetzwerks gespeichert ist,
- Abrufen (S102) eines patientenbezogenen Datensatzes des Patienten aus dem Computernetzwerk,
- Auswählen (S103) einer krankheitsbezogenen Datenmenge aus dem patientenbezogenen Datensatz,
- Bestimmen (S104) mindestens einer der Phasen des krankheitsbezogenen Arbeitsflusses aus dem Satz von krankheitsbezogenen Arbeitsflüssen basierend auf einer ersten krankheitsbezogenen Zuordnungsfunktion, wobei die ausgewählte krankheitsbezogene Datenmenge eine Eingabe der ersten krankheitsbezogenen Zuordnungsfunktion ist,
- Bereitstellen (S107) eines Satzes der Eingabeparameterkategorien,
- Auswählen (S108) einer patientenbezogenen Datenmenge aus dem patientenbezogenen Datensatz, wobei die patientenbezogene Datenmenge mindestens eine Eingabeparameterkategorie umfasst,
- Vergleichen (S109) der patientenbezogenen Datenmenge mit dem bereitgestellten Satz der Eingabeparameterkategorien der Mehrzahl von Computerprogrammen zur Risikobewertung, wodurch eine berechenbare Teilmenge der Mehrzahl von Computerprogrammen zur Risikobewertung bestimmt wird,
- Bestimmen (S105) einer patientenbezogenen Teilmenge der Mehrzahl von Computerprogrammen zur Risikobewertung basierend auf einer zweiten krankheitsbezogenen Zuordnungsfunktion, wobei die bestimmte mindestens eine der krankheitsbezogenen Arbeitsflussphase und der berechenbaren Teilmenge eine Eingabe der zweiten krankheitsbezogenen Zuordnungsfunktion ist, wobei (S110) die patientenbezogene Teilmenge der Mehrzahl von Computerprogrammen zur Risikobewertung durch die bestimmte berechenbare Teilmenge differenziert wird, und
- Anzeigen (S106) der grafischen Benutzeroberfläche, die das Auswahlelement zum Auswählen des einen Computerprogramms zur Risikobewertung aus der patientenbezogenen Teilmenge der Mehrzahl von Computerprogrammen zur Risikobewertung des Patienten enthält, auf der Anzeigeeinheit für den Benutzer, wobei das Anzeigen der grafischen Benutzeroberfläche ein Anzeigen der differenzierten patientenbezogenen Teilmenge der Mehrzahl von Computerprogrammen zur Risikobewertung gemäß ihrer Berechenbarkeit auf der Anzeigeeinheit für den Benutzer umfasst.

2. Verfahren nach Anspruch 1, wobei das Anzeigen der grafischen Benutzeroberfläche ein Identifizieren (S111) solcher Eingabeparameterkategorien der patientenbezogenen Teilmenge der Mehrzahl von Computerprogrammen zur Risikobewertung, die für die Berechenbarkeit fehlen, und Anzeigen der identifizierten fehlenden Eingabeparameterkategorien auf der Anzeigeeinheit für den Benutzer umfasst.

3. Computerimplementiertes Verfahren zum Anzeigen einer grafischen Benutzeroberfläche, die ein Auswahlelement zum Auswählen eines Computerprogramms zur Risikobewertung aus einer patientenbezogenen Teilmenge einer Mehrzahl von Computerprogrammen zur Risikobewertung enthält, auf einer Anzeigeeinheit für einen Benutzer, wobei das Verfahren umfasst:
- Abrufen (S100) eines Satzes von krankheitsbezogenen Arbeitsflüssen, wobei jeder krankheitsbezogene Arbeitsfluss einen klinischen Verlauf und/oder eine Entwicklung eines Patienten hinsichtlich einer spezifischen Krankheit beschreibt und mindestens zwei krankheitsbezogene Phasen umfasst und innerhalb eines Computernetzwerks gespeichert wird, wobei die mindestens zwei krankheitsbezogenen Phasen Teil solch eines klinischen Verlaufs und/oder solch einer Entwicklung eines Patienten hinsichtlich der spezifischen Krankheit sind,
- Abrufen (S101) einer Mehrzahl von Computerprogrammen zur Risikobewertung, wobei jedes Computerprogramm zur Risikobewertung eine Wahrscheinlichkeit eines klinischen Ergebnisses in Abhängigkeit von Werten in Eingabeparameterkategorien vorhersagt und innerhalb des Computernetzwerks gespeichert ist,
- Abrufen (S102) eines patientenbezogenen Datensatzes des Patienten aus dem Computernetzwerk,
- Auswählen (S103) einer krankheitsbezogenen Datenmenge aus dem patientenbezogenen Datensatz,
- Bestimmen (S104) mindestens einer der Phasen des krankheitsbezogenen Arbeitsflusses aus dem Satz von krankheitsbezogenen Arbeitsflüssen basierend auf einer ersten krankheitsbezogenen Zuordnungsfunktion, wobei die ausgewählte krankheitsbezogene Datenmenge eine Eingabe der ersten krankheitsbezogenen Zuordnungsfunktion ist,
- Bereitstellen (S107) eines Satzes der Eingabeparameterkategorien,
- Auswählen (S108) einer patientenbezogenen Datenmenge aus dem patientenbezogenen Datensatz, wobei die patientenbezogene Datenmenge mindestens eine Eingabeparameterkategorie umfasst,
- Vergleichen (S109) der patientenbezogenen Datenmenge mit dem bereitgestellten Satz der Eingabeparameterkategorien der Mehrzahl von Computerprogrammen zur Risikobewertung, wodurch eine berechenbare Teilmenge der Mehrzahl von Computerprogrammen zur Risikobewertung bestimmt wird,
- Bestimmen (S105) einer patientenbezogenen Teilmenge der Mehrzahl von Computerprogrammen zur Risikobewertung basierend auf einer zweiten krankheitsbezogenen Zuordnungsfunktion, wobei die bestimmte mindestens eine der krankheitsbezogenen Arbeitsflussphasen und die berechenbare Teilmenge eine Eingabe der zweiten krankheitsbezogenen Zuordnungsfunktion ist, wobei (S112) die patientenbezogene Teilmenge der Mehrzahl von Computerprogrammen zur Risikobewertung mit der bestimmten berechenbaren Teilmenge zusammengeführt wird, wobei das Zusammenführen eine UND-Operation umfasst und wobei eine berechenbare patientenbezogene Teilmenge der Mehrzahl von Computerprogrammen zur Risikobewertung bestimmt wird, und
- Anzeigen (S106) der grafischen Benutzerschnittstelle, die das Auswahlelement zum Auswählen des Computerprogramms zur Risikobewertung aus der berechenbaren patientenbezogenen Teilmenge der Mehrzahl von Computerprogrammen zur Risikobewertung des Patienten enthält, auf der Anzeigeeinheit für den Benutzer.

4. Verfahren nach einem der vorhergehenden Ansprüche,
- wobei die erste krankheitsbezogene Zuordnungsfunktion durch Trainieren eines Systems für maschinelles Lernen basierend auf einem ersten Satz von Trainingsdaten bestimmt wird und/oder
- wobei die zweite krankheitsbezogene Zuordnungsfunktion durch Trainieren des Systems für maschinelles Lernen basierend auf einem zweiten Satz von Trainingsdaten bestimmt wird.

5. Verfahren nach Anspruch 4, wobei das System für maschinelles Lernen und/oder die erste krankheitsbezogene Zuordnungsfunktion und/oder die zweite krankheitsbezogene Zuordnungsfunktion auf einem künstlichen neuronalen Netzwerk basieren.

6. Verfahren nach Anspruch 5, wobei das künstliche neuronale Netzwerk ein neuronales Faltungsnetzwerk umfasst.

7. Datenverarbeitungseinheit, umfassend:
- einen Prozessor,
- eine Netzwerkschnittstelle,
- die Anzeigeeinheit,
wobei die Datenverarbeitung zum Durchführen des Verfahrens nach einem der Ansprüche 1 bis 6 konfiguriert ist.

8. Computerprogrammprodukt, umfassend ein Computerprogramm, wobei das Computerprogramm in eine Speichereinheit eines Datenverarbeitungssystems geladen werden kann und Programmcodeabschnitte umfasst, um das Datenverarbeitungssystem zum Ausführen des Verfahrens nach einem der Ansprüche 1 bis 6 zu veranlassen, wenn das Computerprogramm im Datenverarbeitungssystem ausgeführt wird.

9. Computerlesbares Medium, auf dem Programmcodeabschnitte eines Computerprogramms gespeichert sind, wobei die Programmcodeabschnitte in ein Datenverarbeitungssystem geladen und/oder von diesem ausgeführt werden können, um das Datenverarbeitungssystem zum Ausführen des Verfahrens nach einem der Ansprüche 1 bis 6 zu veranlassen, wenn die Programmcodeabschnitte im Datenverarbeitungssystem ausgeführt werden.

## Revendications

1. Procédé mis en œuvre par ordinateur d'affichage d'une interface d'utilisateur graphique, qui contient un élément de sélection pour sélectionner un programme informatique d'évaluation du danger parmi un sous-ensemble se rapportant à un patient d'une pluralité de programmes informatiques d'évaluation du danger sur une unité d'affichage pour un utilisateur, le procédé comprenant :
- rechercher (S100) un ensemble de flux de travail se rapportant à une maladie, chaque flux de travail se rapportant à une maladie décrivant une voie clinique et/ou une évolution du patient en ce qui concerne une maladie particulière et comprenant au moins deux stades se rapportant à une maladie et étant mis en mémoire dans un réseau informatique, les au moins deux stades se rapportant à une maladie faisant partie d'une telle voie clinique et/ou évolution d'un patient en ce qui concerne ladite maladie particulière,
- rechercher (S101) une pluralité de programmes informatiques d'évaluation du danger, chaque programme informatique d'évaluation du danger prévoyant une probabilité d'une issue clinique en fonction de valeurs dans des catégories de paramètres d'entrée et étant mis en mémoire dans le réseau informatique,
- rechercher (S102) un enregistrement de données se rapportant à un patient du patient dans le réseau informatique,
- sélectionner (S103) un ensemble de données se rapportant à une maladie dans l'enregistrement de données se rapportant à un patient,
- déterminer (S104) au moins l'un des stades du flux de travail se rapportant à une maladie dans l'ensemble des flux de travail se rapportant à une maladie sur la base d'une première fonction d'implantation se rapportant à une maladie, l'ensemble de données sélectionné se rapportant à une maladie étant une entrée de la première fonction d'implantation se rapportant à une maladie,
- se procurer (S107) un ensemble desdites catégories de paramètres d'entrée,
- sélectionner (S108) un ensemble de données se rapportant à un patient dans l'enregistrement de données se rapportant à un patient, dans lequel l'ensemble de données se rapportant à un patient comprend au moins une catégorie de paramètres d'entrée,
- comparer (S109) l'ensemble de données se rapportant à un patient à l'ensemble procuré des catégories de paramètres d'entrée de la pluralité de programmes informatiques d'évaluation du danger, un sous-ensemble calculable de la pluralité de programmes informatiques d'évaluation du risque étant ainsi déterminé,
- déterminer (S105) un sous-ensemble se rapportant à un patient de la pluralité de programmes informatiques d'évaluation du danger sur la base d'une deuxième fonction d'implantation se rapportant à une maladie, le au moins un stade de flux de travail se rapportant à une maladie et le sous-ensemble calculable étant une entrée de la deuxième fonction d'implantation se rapportant à une maladie, dans lequel (S110) le sous-ensemble se rapportant à un patient de la pluralité de programmes informatiques d'évaluation du danger est différencié par le sous-ensemble calculable déterminé, et
- afficher (S106) l'interface d'utilisateur graphique, qui contient l'élément de sélection, pour sélectionner ledit un programme informatique d'évaluation du danger dans le sous-ensemble se rapportant à un patient de la pluralité de programmes informatiques d'évaluation du danger du patient sur l'unité d'affichage pour l'utilisateur, dans lequel l'affichage de l'interface d'utilisateur graphique comprend afficher le sous-ensemble différencié se rapportant à un patient de la pluralité de programmes informatiques d'évaluation du danger suivant leur calculabilité sur l'unité d'affichage pour l'utilisateur.

2. Procédé suivant la revendication 1, dans lequel l'affichage de l'interface d'utilisateur graphique comprend identifier (S111), telles catégories de paramètres d'entrée du sous-ensemble se rapportant à un patient de la pluralité de programmes informatiques d'évaluation du danger, manquant de calculabilité, et afficher les catégories de paramètres d'entrée identifiées manquantes sur l'unité d'affichage pour l'utilisateur.

3. Procédé mis en œuvre par ordinateur d'affichage d'une interface d'utilisateur graphique, qui contient un élément de sélection pour sélectionner un programme informatique d'évaluation du danger, parmi un sous-ensemble se rapportant à un patient d'une pluralité de programmes informatiques d'évaluation du danger sur une unité d'affichage pour un utilisateur, le procédé comprenant :
- rechercher (S100) un ensemble de flux de travail se rapportant à une maladie, chaque flux de travail se rapportant à une maladie décrivant une voie clinique et/ou une évolution du patient en ce qui concerne une maladie particulière et comprenant au moins deux stades se rapportant à une maladie et étant mis en mémoire dans un réseau informatique, les au moins deux stades se rapportant à une maladie faisant partie d'une telle voie clinique et/ou évolution d'un patient en ce qui concerne ladite maladie particulière,
- rechercher (S101) une pluralité de programmes informatiques d'évaluation du danger, chaque programme informatique d'évaluation du danger prévoyant une probabilité d'une issue clinique en fonction de valeurs dans des catégories de paramètres d'entrée et étant mis en mémoire dans le réseau informatique,
- rechercher (S102) un enregistrement de données se rapportant à un patient du patient dans le réseau informatique,
- sélectionner (S103) un ensemble de données se rapportant à une maladie dans l'enregistrement de données se rapportant à un patient,
- déterminer (S104) au moins l'un des stades du flux de travail se rapportant à une maladie dans l'ensemble des flux de travail se rapportant à une maladie sur la base d'une première fonction d'implantation se rapportant à une maladie, l'ensemble de données sélectionné se rapportant à une maladie étant une entrée de la première fonction d'implantation se rapportant à une maladie,
- se procurer (S107) un ensemble desdites catégories de paramètres d'entrée,
- sélectionner (S108) un ensemble de données se rapportant à un patient dans l'enregistrement de données se rapportant à un patient, dans lequel l'ensemble de données se rapportant à un patient comprend au moins une catégorie de paramètres d'entrée,
- comparer (S109) l'ensemble de données se rapportant à un patient à l'ensemble procuré des catégories de paramètres d'entrée de la pluralité de programmes informatiques d'évaluation du danger, un sous-ensemble calculable de la pluralité de programmes informatiques d'évaluation du risque étant ainsi déterminé,
- déterminer (S105) un sous-ensemble se rapportant à un patient de la pluralité de programmes informatiques d'évaluation du danger sur la base d'une deuxième fonction d'implantation se rapportant à une maladie, le au moins un stade de flux de travail déterminé se rapportant à une maladie et le sous-ensemble calculable étant une entrée de la deuxième fonction d'implantation se rapportant à une maladie, dans lequel (S112) le sous-ensemble se rapportant à un patient de la pluralité de programmes informatiques d'évaluation du danger est fusionné avec le sous-ensemble calculable déterminé, dans lequel fusionner comprend une opération ET et dans lequel un sous-ensemble calculable se rapportant à un patient de la pluralité de programmes informatiques d'évaluation du danger est déterminé, et
- afficher (S106) l'interface d'utilisateur graphique, qui contient l'élément de sélection, pour sélectionner ledit un programme informatique d'évaluation du danger dans le sous-ensemble calculable se rapportant à un patient de la pluralité de programmes informatiques d'évaluation du danger du patient sur l'unité d'affichage pour l'utilisateur.

4. Procédé suivant l'une des revendications précédentes,
- dans lequel on détermine la première fonction d'implantation se rapportant à une maladie en faisant subir un apprentissage à un système d'enseignement automatique sur la base d'un premier ensemble de données d'apprentissage, et/ou
- dans lequel on détermine la deuxième fonction d'implantation se rapportant à une maladie en faisant subir un apprentissage au système automatique d'enseignement sur la base d'un deuxième ensemble de données d'apprentissage.

5. Procédé suivant la revendication 4, dans lequel le système d'enseignement automatique et/ou la première fonction d'implantation se rapportant à une maladie et/ou la deuxième fonction d'implantation se rapportant à une maladie reposent sur un réseau neuronal artificiel.

6. Procédé suivant la revendication 5, dans lequel le réseau neuronal artificiel comprend un réseau neuronal de convolution.

7. Unité de traitement de données, comprenant :
- un processeur,
- une interface de réseau,
- l'unité d'affichage,
dans lequel le traitement de données est configuré pour effectuer le procédé suivant l'une quelconque des revendications 1 à 6.

8. Produit de programme d'ordinateur comprenant un programme informatique, le programme informatique pouvant être chargé dans une unité de mémoire d'un système de traitement de données, comprenant des parties de code de programme pour faire que le système de traitement de données exécute le procédé suivant l'une quelconque des revendications 1 à 6, lorsque le programme informatique est exécuté dans ledit système de traitement de données.

9. Support, déchiffrable par ordinateur, sur lequel des parties de code de programme d'un programme informatique sont sauvegardées, lesdites parties de code de programme pouvant être chargées et/ou exécutables dans un système de traitement de données pour faire que le système de traitement de données exécute le procédé suivant l'une quelconque des revendications 1 à 6, lorsque les parties de code de programme sont exécutées dans ledit système de traitement de données.
